# EUROPÄISCHE PATENTANMELDUNG

(11) **EP 4 491 271 A1**
(43) Veröffentlichungstag der Anmeldung: **15.01.2025**
(21) Anmeldenummer: 23185098.3
(22) Anmeldetag: 12.07.2023
(51) Int. Cl.: B01L 3/00, G01N 33/543

(54) **VORRICHTUNG ZUM VISUELLEN NACHWEIS VON ANALYTEN IN EINER FLÜSSIGEN PROBE**

(71) Anmelder: Protzek Biotec GmbH, 4415 Lausen (CH)
(72) Erfinder: PROTZEK, Christoph, 79539 Lörrach (DE)
(74) Vertreter: Patentanwälte Dörner & Kötter PartG mbB

(57) **Zusammenfassung**

Die Erfindung betrifft eine Vorrichtung zum visuellen Nachweis von Analyten in einer vom menschlichen oder tierischen Organismus stammenden Analysen- oder Flüssigkeitsprobe, umfassend eine aus Karton hergestellte Trägerplatte (2) mit wenigstens einer Teststreifenaufnahme (21), die durch eine in die Trägerplatte (2) eingebrachte Einprägung oder Ausnehmung ausgebildet ist und in der ein mit einem Probeaufnahmeabschnitt (42) und einem Testabschnitt (44) versehener Teststreifen (4) angeordnet ist, wobei die wenigstens eine Teststreifenaufnahme (21) zumindest bereichsweise über eine nicht lösbar mit der Trägerplatte (2) verbundene, aus Karton hergestellte Deckschicht (3) verschlossen ist, die im Bereich des Testabschnitts (44) des Teststreifens (4) der wenigstens einen Teststreifenaufnahme (21) wenigstens ein Probeaufgabenfenster (32) und wenigstens ein Kontrollfenster (33) aufweist, wobei die Deckschicht (3) an ihrer der Trägerplatte (2) zugewandten Seite wenigstens einen hervorstehenden Steg (34, 35) aufweist, der sich quer zu dem wenigstens einen Teststreifen (4) über diesen hinweg erstreckt.

## Beschreibung

Die Erfindung betrifft eine Vorrichtung zum visuellen Nachweis von Analyten in einer vom menschlichen oder tierischen Organismus stammenden Analysen-oder Flüssigkeitsprobe nach dem Oberbegriff des Patentanspruchs 1. Die Erfindung betrifft weiterhin ein Verfahren zur Herstellung einer solchen Vorrichtung nach dem Patentanspruch 9.

Vorrichtungen zum Nachweis von Analyten in einer flüssigen Probe, zum Beispiel von Drogen, Medikamenten oder Antikörpern als Zeichen für bestimmte Erkrankungen in einer Körperflüssigkeit oder Feststellungen im Sinne von Konzentrationsangaben sind in unterschiedlichen Ausführungen bekannt. Besonderes Interesse besteht an solchen Geräten, die auch außerhalb eines Labors eingesetzt werden können. Diese müssen tragbar und leicht zu bedienen sein sowie in kurzer Zeit ein zuverlässiges Ergebnis liefern. Insbesondere zur Durchführung von Tests auf Drogenkonsum oder Medikamentenmissbrauch oder dergleichen durch die Polizei ist eine Vorrichtung erforderlich, die auch im Freien bei unterschiedlichen wechselnden Lichtverhältnissen, Wetterverhältnissen und Temperaturen einen derartigen Test ermöglicht. In der DE 20 2014 002 369 U1 wird hierzu ein Gerät vorgeschlagen, in das eine Testkassette einführbar ist und dass mit einer Lichtquelle versehen ist, mittels derer die untere Wand der aus einem lichtdurchlässigen Kunststoff bestehenden Testkassette sowie den Testbereich des in der Testkassette angeordneten Teststreifens durchstrahlt und das Testergebnis ausleuchtet. Die Testkassette ist zur Durchführung eines lateral flow immuno-assays, Aptamer basierter Assay oder Encym immuno assay (EIA) ausgelegt und besteht aus einem unteren Deckel und einem oberen Deckel. Eingeschlossen von diesen beiden Deckeln enthält die Testkassette einen Teststreifen, der innerhalb einer Aufnahme in Position gehalten wird. In dem oberen Deckel befindet sich über dem Probenaufnehmer des Teststreifens eine Einfüllöffnung, durch die eine zu untersuchende flüssige Probe eingegeben werden kann. Über den mittleren Testbereich des Teststreifens befindet sich ein Kontrollfenster, durch welches das Testergebnis abgelesen werden kann. Derartige Kassetten haben sich im mobilen Einsatz bewährt. Nachteilig an den vorbekannten Kassetten ist jedoch, dass dessen Herstellung aufwendig ist und diese relativ viel Platz benötigen. Zudem ist durch die vorbekannten Kunststoffkassetten ein erhebliches Abfallaufkommen verursacht.

Vor diesem Hintergrund wird in der EP 3287785 A1 eine Vorrichtung vorgestellt, die im Wesentlichen aus Karton hergestellt ist. Hierbei ist wenigstens ein Teststreifen von einer Trägerplatte aufgenommen auf die eine Deckschicht aufgebracht ist, die im Bereich des Testabschnitts des wenigstens einen Teststreifens wenigstens ein Probeaufgabenfenster und wenigstens ein Kontrollfenster aufweist. Diese Vorrichtung, die sich dadurch zeichnet, dass sie äußerst platzsparend aufgebaut, kostengünstig herzustellen und umweltfreundlich zu entsorgen ist, hat sich in der Praxis bewährt.

Der vorliegenden Erfindung liegt die Aufgabe zu Grunde, die Effizienz der in der EP 3287785 A1 beschriebenen Vorrichtung weiter zu verbessern. Gemäß der Erfindung wird diese Aufgabe durch eine Vorrichtung mit den Merkmalen des Patentanspruchs 1 gelöst.

Mit der Erfindung ist eine Vorrichtung der vorstehenden Art bereitgestellt, deren Effizienz verbessert ist. Dadurch, dass die aus Karton hergestellte Deckschicht an ihrer der Trägerplatte zugewandten Seite wenigstens einen hervorstehenden Steg aufweist, der sich quer zu dem wenigstens einen Teststreifen über diesen hinweg erstreckt, wird der Querschnitt des Teststreifens partiell vermindert, wodurch die Fließgeschwindigkeit einer aufgegebenen Probe in dem Testreifen erhöht wird. Dabei ist der wenigstens eine Steg bevorzugt zwischen dem Probeaufgabenfenster und dem Kontrollfenster angeordnet.

Unter dem Begriff Karton ist vorliegend ein flächiger, im Wesentlichen aus Fasern meist pflanzlicher Herkunft bestehender Werkstoff, zu verstehen, wobei hierunter auch Vollpappe und Wellpappe zu subsummieren ist.

In Weiterbildung der Erfindung sind zwei Stege beabstandet zueinander angeordnet. Hierdurch ist die Fließgeschwindigkeit einer aufgegebenen Probe in dem Teststreifen weiter verbessert.

In Ausgestaltung der Erfindung ist der wenigstens eine Steg durch eine in die Deckschicht eingebrachte Einprägung gebildet. Hierdurch ist eine einfache und kostengünstige Herstellung ermöglicht.

In weiterer Ausgestaltung der Erfindung sind wenigstens zwei parallel zueinander angeordnete, jeweils einen Teststreifen aufnehmende Teststreifenaufnahmen angeordnet, die an einem Ende in einem gemeinsamen Reservoir münden, in dem die Probeaufnahmeabschnitte der Teststreifen angeordnet sind und über dem das wenigstens eine Probeaufgabefenster der Deckschicht positioniert ist. Hierdurch ist eine gleichzeitige Probeaufgabe auf alle in dem Reservoir mündenden Probeaufnahmeabschnitten der Teststreifen ermöglicht. Über die Ausgestaltung des Reservoirs ist zudem eine ausreichende Zufuhr an Flüssigkeitsprobe gewährleistet, sowie auch eine homogene Verteilung der Flüssigkeit auf dem vorzugsweise mit einer Separationsfolie (Probenvlies) versehenen Probeaufnahmeabschnitt der Teststreifen. Alternativ können die Teststreifenaufnahmen auch beabstandet zu dem Reservoir münden, wobei die Teststreifen die Teststreifenaufnahmen überragen und mit ihren Probeaufnahmeabschnitte in dem Reservoir angeordnet sind.

In Weiterbildung der Erfindung ist im Bereich des Probeaufgabefensters zwischen Deckschicht und Teststreifen ein Probeaufnahmevlies angeordnet. Hierdurch ist eine gleichmäßige Beaufschlagung der Teststreifen mit der Flüssigkeitsprobe bewirkt.

In Ausgestaltung der Erfindung weist die Deckschicht eine nach außen, der Trägerplatte entgegengerichtete Einwölbung auf, in der das wenigstens eine Probeaufgabefenster eingebracht ist. Hierdurch ist ein Aufnahmeraum ausgebildet, durch den das Flüssigkeitsaufnahmevolumen vergrößert ist.

In weiterer Ausgestaltung der Erfindung sind die Trägerplatte und die Deckschicht aus einem gemeinsamen, entlang einer Knicklinie umgeknickten Kartonzuschnitt gebildet. Hierdurch ist eine kostengünstige Herstellung ermöglicht. Dabei können die Trägerplatte mit den dort angeordneten Teststreifenaufnahmen, Stegen und Reservoir und die Deckschicht mit den in dieser angeordneten Probeaufgabe- und Kontrollfenstern gemeinsam in einem Stanz-Prägeprozess hergestellt werden. Die Deckschicht kann nach Einbringen des wenigstens einen Teststreifens in die wenigstens eine Teststreifenaufnahme einfach umgeknickt und mit der Trägerplatte verklebt werden.

In Weiterbildung der Erfindung ist zwischen dem wenigstens einen Probeaufgabefenster und der wenigstens einen Teststreifenaufnahme eine Perforationslinie vorhanden, die sich über die gesamte Breite der Vorrichtung erstreckt. Hierdurch ist der sich über den Probeaufnahmeabschnitt des wenigstens einen Teststreifens erstreckende Abschnitt der Trägerplatte mit dem auf diesem angeordneten Abschnitt der Deckschicht bei Bedarf abgetrennt werden, wonach die Vorrichtung für einen Tauchtest einsetzbar ist.

Der vorliegenden Erfindung liegt weiterhin die Aufgabe zu Grunde, ein Verfahren zur kostengünstigen Herstellung einer Vorrichtung der vorbekannten Art bereitzustellen, deren Effizienz verbessert ist. Gemäß der Erfindung wird diese Aufgabe durch ein Verfahren mit den Merkmalen des Patentanspruchs 9 gelöst. Dadurch, dass die Vorrichtung durch einen einzigen Stanz-Prägeprozess mit nachfolgendem Einlegen der Teststreifen und umknicken und Verkleben der Deckschicht auf der Trägerplatte hergestellt wird, ist ein sehr effizienter und kostengünstiger Herstellungsprozess erzielt.

In Weiterbildung der Erfindung wird in dem Stanz-Prägeprozess zusätzlich in die Deckschicht das wenigstens eine Probeaufgabefenster umgebend ein nach außen, der Trägerplatte entgegengerichteter ausgewölbter Bereich eingeprägt. Hierdurch ist ein Aufnahmeraum erzielt, durch den das Aufnahmevolumen für eine Probenflüssigkeit vergrößert wird.

Andere Weiterbildungen und Ausgestaltungen der Erfindung sind in den übrigen Unteransprüchen angegeben. Ausführungsbeispiele der Erfindung sind in den Zeichnungen dargestellt und werden nachfolgend im Einzelnen beschrieben. Es zeigen:

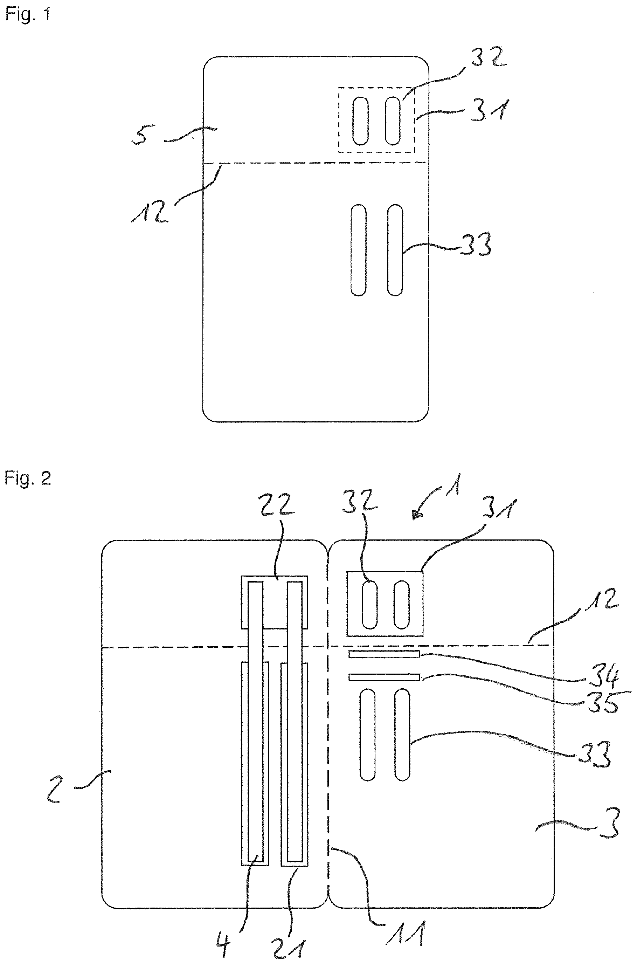

- Figur 1: die schematische Darstellung einer Vorrichtung zum visuellen Nachweis von Analyten in einer flüssigen Probe;
- Figur 2: die schematische Darstellung der Vorrichtung aus Figur 1 in aufgeklapptem Zustand und
- Figur 3: die schematische Darstellung eines Teststreifen der Vorrichtung aus Figur 1.

Die als Ausführungsbeispiel gewählte Vorrichtung zum visuellen Nachweis von Analyten in einer flüssigen Probe umfasst einen Kartonzuschnitt 1, der mit einer ersten, mittig angeordneten Perforationslinie 11 versehen ist, durch die ein erster, eine Trägerplatte 2 bildender Abschnitt und ein zweiter, eine Deckschicht 3 bildender Abschnitt gebildet sind, die über die erste Perforationslinie 11 schwenkbar miteinander verbunden sind. Anstelle der ersten Perforationslinie 11 kann auch eine Knickfalz angeordnet sein. Im Ausführungsbeispiel ist der Kartonzuschnitt 1 mit einem wasserdichten, umweltgerechten Lack versehen, wodurch ein Eindringen von Probenflüssigkeit verhindert ist.

In die Trägerplatte 2 sind zwei Teststreifenaufnahmen 21 zur Aufnahme jeweils eines Teststreifens 4 eingeprägt. Beabstandet zu den Teststreifenaufnahmen 21 ist ein im Ausführungsbeispiel im Wesentlichen rechteckförmiges Reservoir 22 eingeprägt. Zwischen den beiden Teststreifenaufnahmen 21 und dem Reservoir 22 ist orthogonal zu der ersten Perforationslinie 11 eine zweite Perforationslinie 12 angeordnet, die sich über den gesamten Kartonzuschnitt 1 erstreckt.

In die Deckschicht 3 ist beabstandet zu dem Reservoir 22 der Trägerplatte 2 eine im Wesentlichen rechteckig ausgebildete Einwölbung 31 eingeprägt, in die beabstandet zueinander zwei Probenaufgabefenster 32 eingebracht sind. Die Einwölbung 32 ist derart dimensioniert, dass sie im zusammengeklappten Zustand des Kartonzuschnitts 1, in dem die Deckschicht 3 auf der Trägerplatte 2 aufliegt, das Reservoir 22 vollständig überspannt.

Beabstandet zu der Einwölbung 32 sind parallel beabstandet zueinander zwei länglich ausgebildete Kontrollfenster 33 in die Deckschicht 3 eingebracht. Die Kontrollfenster 33 sind derart positioniert, dass sie sich im zusammengeklappten Zustand des Kartonzuschnitts 1 jeweils über dem Testabschnitt 44 eines in einer Teststreifenaufnahme 21 angeordneten Teststreifens 4 befindet.

Zwischen der Einwölbung 32 und dem Kontrollfenstern 33 sind zwei Stege 34, 35 angeordnet, die im Ausführungsbeispiel durch Einprägungen gebildet sind. Alternativ können die Stege 34, 35 auch durch einen Materialauftrag auf die Deckschicht 3 hergestellt sein. Die beiden Stege 34, 35, die vorzugsweise gerade ausgebildet und parallel zueinander angeordnet sind, sind derart positioniert, dass im zusammengeklappten Zustand des Kartonzuschnitts 1 ein erster Steg 34 zwischen der Teststreifenaufnahme 21 und der zweiten Perforationslinie 12 und der zweite Steg 35 im Bereich der Teststreifenaufnahmen 21 auf den von diesen aufgenommenen Teststreifen 4 aufliegen, wodurch der Querschnitt der Teststreifen 4 partiell verringert wird. An Stelle der Stege 34, 35 können auch partielle Aufwölbungen angeordnet sein, die auf dem Teststreifen 4 zu deren partieller Querschnittsverjüngung an den vorstehend genannten Bereichen aufliegen. Insoweit muss ein "Steg" vorliegend nicht zwingend eine längliche Ausgestaltung haben.

In einer weiteren Ausführungsform können sich die beiden Teststreifenaufnahmen 21 auch bis in das Reservoir 22 hinein erstrecken. In diesem Fall sind beide Stege 34, 35 im zusammengeklappten Zustand des Kartonzuschnitts 1 im Bereich der Teststreifenaufnahmen 21 angeordnet.

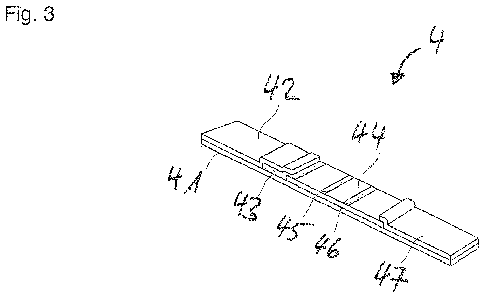

Der Teststreifen 4 ist in Figur 3 im Stufenschnitt dargestellt. Der Teststreifen 4 weist eine Trägerfolie 41 auf, auf der an einem Ende ein Probeaufnahmeabschnitt 42 angeordnet ist. Unmittelbar benachbart und teilweise unter dem Probeaufnahmeabschnitt 42 befindet sich auf der Trägerfolie 41 ein Antikörperbelag 43, der Antikörper enthält, die mit Goldpartikeln konjugiert sind und die zu einem festzustellenden Analyten bzw. zu einem Antigen dieses Analyten passen und in eine immunologische Reaktion treten können. An den Antikörperbelagabschnitt 43 schließt sich ein Testabschnitt 44 an, der in Form einer Membran ausgebildet ist, auf den sich beabstandet zueinander eine Testlinie 45 und eine Kontrolllinie 46 befinden, die durch in einem Reagenz für den Nachweis eines bestimmten Analyten enthaltenen Antigen ausgebildet sind und vor Aufgabe einer Probe unsichtbar sind. Der Probenaufnahmeabschnitt 42 des Teststreifens 4 befindet sich unterhalb der Probenaufgabefenster 32 und der Testabschnitt 44 befindet sich unterhalb des Kontrollfensters 33 der Deckschicht 3.

Bei der Durchführung eines lateral flow assays zeigt die Testlinie 45 das Ergebnis des Tests an; die Kontrolllinie 46 gibt an, ob eine ausreichende Probemenge aufgegeben worden ist, also ob der Test valide ist und seine Durchführung sachgemäß erfolgte. An seinem dem Probenaufnahmeabschnitt 42 gegenüberliegenden Ende ist an dem Teststreifen 4 ein Absorberabschnitt 47 angeordnet.

Im montierten Zustand der scheckkartengroßen Vorrichtung ist in jede Teststreifenaufnahme 21 der Trägerplatte 2 ein Teststreifen 4 eingebracht, wobei deren Probeaufnahmeabschnitte 42 in dem Reservoir 22 positioniert sind. Im Ausführungsbeispiel ist die Probeaufnahmeabschnitte 42 der Teststreifen 4 überdeckend ein - nicht dargestelltes - Probeaufnahmevlies angeordnet. Die Deckschicht 3 ist entlang der ersten Perforationslinie 11 umgeklappt und liegt auf der Trägerplatte 2 auf, mit der sie verklebt ist. Durch die Einwölbung 31 ist das Reservoir 22 einfassend ein vergrößerter Probeaufnahmeraum gebildet.

Vor der Durchführung eines Tests sind keine Linien 45, 46 ersichtlich. Bei der Durchführung eines Tests wird ein Probevolumen einer Probeflüssigkeit durch ein Probeaufgabefenster 32 der Deckschicht 3 auf den Probenaufnahmeabschnitt 42 eines jeden Teststreifens 4 aufgegeben. Durch Kapilarwirkung fließt die Probenflüssigkeit in Richtung Testabschnitt 44. Sie kommt dabei zunächst mit dem Antikörperbelagabschnitt 43 in Kontakt, in dem sich mit Goldpartikeln konjugierte Antikörper zum Antigen eines festzustellenden Analyten in standardisierter (dosierter) Menge befinden. Die Antikörper weisen eine für verschiedene Analyten (Drogen) unterschiedliche Bindungsaktivität auf. Die mit Goldpartikeln konjugierten Antikörper werden durch die Probenflüssigkeit mitgenommen, welche den Testabschnitt 44 durchfließt.

Enthält die Probenflüssigkeit nicht den festzustellenden Analyten, so werden beim Durchfließen des Testsabschnitts 44 die dort im Bereich der Testlinie 45 befindlichen Antigene des Analyten durch die von der Probenflüssigkeit mitgenommenen Antikörper gebunden und es bildet sich eine sichtbare Testlinie 45 heraus, die durch die mit den Antikörpern konjugierten Goldpartikel eine rote oder braune Farbe erhält. Enthält die Probenflüssigkeit den festzustellenden Analyten, so werden dessen Antigene bereits durch die Antikörper im Bereich des Antikörperbelagabschnitts 43 des Teststreifens 4 gebunden und es kann im Testabschnitt 44 nicht mehr zu einer Anlagerung kommen, weshalb sich keine sichtbare Testlinie 45 herausbildet. Die Kontrolllinie 46 zeigt an, ob der Test valide ist.

Im Bereich zwischen dem Reservoir 22 bzw. dem Probenaufgabefenster 32 und den Teststreifenaufnahmen 21 ist zwischen der Trägerplatte 2 und der Deckschicht 3 kein Kleber vorhanden. Der durch die zweite Perforationslinie 12 begrenzte, das Reservoir 22 bzw. das Probenaufgabefenster 32 aufweisende Abschnitt 5 kann entlang der zweiten Perforationsline 12 abgetrennt werden, wonach die Probeaufnahmeabschnitte 42 der Teststreifen 4 freigelegt sind. Die Vorrichtung kann auf diese Weise auch für einen Tauchtest eingesetzt werden.

Zur Herstellung der erfindungsgemäßen Vorrichtung werden in einem einzigen Stanz-Prägeprozess die Trägerplatte 2 und die Deckplatte 3 nebeneinanderliegend einteilig aus einem Karton ausgestanzt, wobei zwischen Trägerplatte 2 und Deckplatte 3 eine Knicklinie eingeprägt wird. Innerhalb des Prozesses werden weiterhin in die Trägerplatte 2 die Teststreifenaufnahmen 21 eingeprägt, in der Deckschicht werden die Stege 34, 35 sowie die Einwölbung 31 eingeprägt und die Probeaufgabefenster 32 und die Kontrollfenster 33 werden ausgestanzt. Sodann wird in die Teststreifenaufnahmen 21 jeweils ein Teststreifen 4 eingebracht. Nachfolgend wird die Deckschicht 3 entlang der als Knicklinie fungierenden ersten Perforationslinie 11 umgeknickt, über die Teststreifen 4 auf die Trägerplatte 2 gelegt und mit dieser verklebt.

## Patentansprüche

1. Vorrichtung zum visuellen Nachweis von Analyten in einer vom menschlichen oder tierischen Organismus stammenden Analysen- oder Flüssigkeitsprobe, umfassend eine aus Karton hergestellte Trägerplatte (2) mit wenigstens einer Teststreifenaufnahme (21), die durch eine in die Trägerplatte (2) eingebrachte Einprägung oder Ausnehmung ausgebildet ist und in der ein mit einem Probeaufnahmeabschnitt (42) und einem Testabschnitt (44) versehener Teststreifen (4) angeordnet ist, wobei die wenigstens eine Teststreifenaufnahme (21) zumindest bereichsweise über eine nicht lösbar mit der Trägerplatte (2) verbundene, aus Karton hergestellte Deckschicht (3) verschlossen ist, die im Bereich des Testabschnitts (44) des Teststreifens (4) der wenigstens einen Teststreifenaufnahme (21) wenigstens ein Probeaufgabenfenster (32) und wenigstens ein Kontrollfenster (33) aufweist, **dadurch gekennzeichnet, dass** die Deckschicht (3) an ihrer der Trägerplatte (2) zugewandten Seite wenigstens einen hervorstehenden Steg (34, 35) aufweist, der sich quer zu dem wenigstens einen Teststreifen (4) über diesen hinweg erstreckt.

2. Vorrichtung nach Anspruch 1, **dadurch gekennzeichnet, dass** der wenigstens eine Steg (34, 35) durch eine in die Deckschicht (3) eingebrachte Einprägung gebildet ist.

3. Vorrichtung nach Anspruch 1 oder 2, **dadurch gekennzeichnet, dass** der wenigstens eine Steg (34, 35) zwischen dem Probeaufgabenfenster (32) und dem Kontrollfenster (33) angeordnet ist.

4. Vorrichtung nach einem der vorgenannten Ansprüche, **dadurch gekennzeichnet, dass** wenigstens zwei parallel zueinander angeordnete, jeweils einen Teststreifen (4) aufnehmende Teststreifenaufnahmen (21) angeordnet sind, die an einem Ende in einem gemeinsamen Reservoir (22) münden, in dem die Probeaufnahmeabschnitte (42) der Teststreifen (4) angeordnet sind und über dem wenigstens ein Probeaufgabefenster (33) der Deckschicht (3) positioniert ist.

5. Vorrichtung nach Anspruch 4, **dadurch gekennzeichnet, dass** im Bereich des Probeaufgabefensters (32) zwischen Deckschicht (3) und Teststreifen (4) ein Probeaufnahmevlies angeordnet ist.

6. Vorrichtung nach einem der vorgenannten Ansprüche, **dadurch gekennzeichnet, dass** das die Deckschicht (3) eine nach außen, der Trägerplatte (2) entgegengerichtete Einwölbung (31) aufweist, in der das wenigstens eine Probeaufgabefenster (32) eingebracht ist.

7. Vorrichtung nach einem der vorgenannten Ansprüche, **dadurch gekennzeichnet, dass** die Trägerplatte (2) und die Deckschicht (3) aus einem gemeinsamen, entlang einer Knicklinie umgeknickten Kartonzuschnitt (1) gebildet sind.

8. Vorrichtung nach einem der vorgenannten Ansprüche, **dadurch gekennzeichnet, dass** zwischen dem wenigstens einen Probeaufgabefenster (32) und der wenigstens einen Teststreifenaufnahme (21) eine Perforationslinie (12) vorhanden ist, die sich über die gesamte Breite der Vorrichtung erstreckt.

9. Verfahren zur Herstellung einer Vorrichtung nach einem der vorgenannten Ansprüche, **dadurch gekennzeichnet, dass** in einem einzigen Stanz-Prägeprozess die Trägerplatte (2) und die Deckplatte (3) nebeneinanderliegend einteilig aus einem Karton ausgestanzt werden, zwischen Trägerplatte (2) und Deckplatte (3) eine Knicklinie eingeprägt wird, in die Trägerplatte (2) wenigstens eine Teststreifenaufnahme (21) eingeprägt wird, in der Deckschicht (2) der wenigstens eine Steg (34, 35) eingeprägt und die Probeaufgabe- sowie Kontrollfenster (32, 33) ausgestanzt werden, wonach in die Teststreifenaufnahmen (21) jeweils ein Teststreifen (4) eingebracht wird und nachfolgend die Deckschicht (3) entlang der Knicklinie umgeknickt und über den wenigstens einen Teststreifen (4) auf die Trägerplatte (2) gelegt und mit dieser verklebt wird.

10. Verfahren nach Anspruch 10, **dadurch gekennzeichnet, dass** in dem Stanz-Prägeprozess in die Deckschicht (3) das wenigstens eine Probeaufgabefenster (32) umgebend eine nach außen, der Trägerplatte (2) entgegengerichtete Einwölbung (31) eingeprägt wird.
